# EUROPÄISCHE PATENTSCHRIFT

(11) **EP 1 863 929 B1**
(45) Veröffentlichungstag und Bekanntmachung des Hinweises auf die Patenterteilung: **21.12.2016**
(21) Anmeldenummer: 06725099.3
(22) Anmeldetag: 16.03.2006
(51) Int. Cl.: C12Q 1/68

(54) **VERFAHREN ZUR DETEKTION VON ZIEL-NUKLEINSÄUREN**
METHOD FOR DETECTING TARGET NUCLEIC ACIDS
PROCEDE DESTINE A LA DETECTION D'ACIDES NUCLEIQUES CIBLES

(30) Priorität: 16.03.2005 DE 102005000021
(43) Veröffentlichungstag der Anmeldung: 12.12.2007
(73) Patentinhaber: AJ Roboscreen GmbH, 04129 Leipzig (DE)
(72) Erfinder: ROST, Anne-Katrin, D-04451 Borsdorf (DE)
(74) Vertreter: Wehlan, Helmut
(86) Internationale Anmeldenummer: PCT/EP2006/060786
(87) Internationale Veröffentlichungsnummer: WO 2006/097506

(56) Entgegenhaltungen:
- EP-A- 1 389 638
- WO-A-01/90399
- WO-A-99/42616
- US-A1- 5 565 322
- US-A1- 5 914 230
- CHEN X. ET AL.: "A HOMOGENEOUS, LIGASE-MEDIATED DNA DIAGNOSTIC TEST" GENOME RESEARCH, COLD SPRING HARBOR LABORATORY PRESS, WOODBURY, NY, US, Bd. 8, Nr. 5, Mai 1998 (1998-05), Seiten 549-556, XP000778987 ISSN: 1088-9051 in der Anmeldung erwähnt
- DIDENKO V.V.: "DNA PROBES USING FLUORESCENCE RESONANCE ENERGY TRANSFER (FRET): DESIGNS AND APPLICATIONS" BIOTECHNIQUES, INFORMA LIFE SCIENCES PUBLISHING, WESTBOROUGH, MA, US, Bd. 31, Nr. 5, November 2001 (2001-11), Seiten 1106-1121, XP001082961 ISSN: 0736-6205 in der Anmeldung erwähnt

## Beschreibung

Die Erfindung betrifft ein Verfahren zur Detektion beliebiger Ziel-Nukleinsäuren oder Nukleinsäureanaloga (Targets), bei dem mindestens ein Paar ligand-markierter Oligonukleotide oder Oligonukleotidanaloga (Sonden) oder mindestens ein ligand-markiertes Sondenterzett zum Nachweis genetischer Polymorphismen eingesetzt wird, wobei die upstream Sonde(n) anteilig mit ihrem 5'-Ende an das Target und mit ihrem 3'-Ende anteilig an das 5'-Ende der downstream Sonde hybridisieren und das 3'-Ende der downstream Sonde wiederum anteilig an das Target hybridisiert, so dass im Resultat eine Triplex-Struktur zwischen der Ziel-Nukleinsäure und einem Sondenpaar ausgebildet wird.

Die Erfindung betrifft Sondenpaare und Sondenterzetts zur homogenen Detektion von heteropolymeren Zielsequenzen für den qualitativen und quantitativen "real-time" oder Endpunkt-Nachweis von heteropolymeren Nukleinsäuren oder Nukleinsäureanaloga oder genetischen Polymorphismen vorzugsweise nach deren enzymatischer Vervielfältigung. Mögliche Anwendungsgebiete sind die Molekularbiologie, die qualitative und quantitative Analyse von Genen, Genexpressionen, viralen bzw. mikrobiellen Krankheitserregern, genetisch veränderten Organismen in Lebensmitteln insbesondere für die individualisierte medizinische Diagnostik, die funktionelle Genomanalyse, die klinische Pharmakologie, Pharmakogenetik, Forensik, die Lebensmittel- und Umweltanalytik, sowie der Einsatz für den ultrasensitiven Nachweis von Proteinen mittels Immuno-PCR.

Für die molekulare Diagnostik qualitativer Genveränderungen und Polymorphismen, sowie die quantitative Erregerdiagnostik von insbesondere extrem geringer Analytkonzentrationen werden häufig enzymatische Amplifizierungsverfahren eingesetzt. Beispiele hierfür sind z.B. die Verfahren Polymerasekettenreaktion (PCR, US Patente 4,683,195 A, 4,683,202 A, EP 0 200 362 A1, EP 0 201 184 A1; Hoffmann-La Roche), Ligasekettenreaktion (LCR, Abbott Diagnostics, North Chicago, IL, USA), Strand Displacement Amplification (SDA, Walker et. al. [1993], PCR Methods Appl. 3: 1-6, Becton-Dickinson Research Center) und Transcription-Mediated Amplification (TMA, Gen-Probe Inc., San Diego, CA). Alle genannten Verfahren stellen in vitro Methoden zur exponentiellen Vermehrung (Amplifikation) der zu messenden Ziel-Nukleinsäure dar.

Homogene Assays erlauben es, die *in vitro* Synthese von PCR-Produkten "real-time", d.h. direkt im jeweils verwendeten PCR-Reaktionsgefäß zu messen. Hierfür geeignete Verfahren nach dem derzeitigen Stand der Technik stellen u.a. das auf "Fluorescence resonance energy transfer (FRET)" basierende "Dye-labeled oligonucleotide ligation (DOL)" Verfahren [Chen, X., Livak, K.J. & Kwok, P.Y. Genome Res. 8, 549-556 (1998), Landegren, U., Nilsson, M. & Kwok, P.Y. Genome Res. 8, 769-776 (1998). US 6,027,889; US 6,268,148] das 5'-Exonuklease oder TaqMan-Assay [Holland, P.M., Abramson, R.D., Watson, R. & Gelfand, D.H. Proc.Natl.Acad.Sci.U.S.A. 88, 7276-7280 (1991), Didenko, V.V. Biotechniques 31, 1106-1 (2001); US 5,210,015; US 5,487,972; EP 0 919 565 A2; WO 92/02638] sowie das "Adjacent hybridization probes" oder Dual Probes-Verfahren (US 5,532,129; US 5,565,322, US 5,914,230, EP 1 389 638 A1) dar. Zu den sog. nicht FRETbasierenden Detektionsverfahren gehören beispielsweise die "Molecular beacons" [Kwiatkowski, R.W., Lyamichev, V., de Arruda, M. & Neri, B. Mol.Diagn. 4, 353-364 (1999), US 5,989,823], die "Self priming probes" (US 5,866,336), das LightUp-Verfahren (EP 1 357 185 A1, US 6,461,871 B1) sowie das PCR-unabhängige Invader-Assay [Cooksey, R.C., Holloway, B.P., Oldenburg, M.C., Listenbee, S. & Miller, C.W. Antimicrob. Agents Chemother. 44, 1296-1301 (2000)].

Für Nukleinsäure-Quantifizierungsreaktionen wird sehr häufig das homogene, sehr robuste 5' - 3'-Exonuklease-Assay angewandt. Dieses Verfahren beruht auf einem dem Fachmann bekannten konventionellen PCR-Prinzip, wobei eine gleichzeitig im Ansatz enthaltene, Amplicon-Einzelstrang komplementäre, einzelsträngige DNA-Sonde zwischen den Primerbindungsstellen am Target hybridisiert. Diese am 5'-Ende mit einem Reporter- und am 3'-Ende mit einem Quencher-Farbstoff markierte Sonde wird infolge Primerextension durch die 5' - 3'-Exonuklease Aktivität der Taq-Polymerase hydrolysiert, wobei resultierend der Quench-Effekt aufgehoben und ein der Menge an Target-Produkt proportionales Fluoreszenzsignal erzeugt wird.

Die oben genannten Verfahren beruhen im Wesentlichen auf der Ausbildung von Duplexstrukturen zwischen Zielsequenz und markierter(n) Sonde(n) und haben den Nachteil, dass pro enzymatisch synthetisiertem Amplifikationsprodukt immer nur maximal ein Fluoreszenzmessimpuls generiert werden kann. Ein weiterer Nachteil besteht darin, dass die verwendeten Sondenformate entweder für das Dual probes oder 5' - 3'-Exonuklease-Assay, aber niemals gleichzeitig für beide Detektionsformate geeignet sind. Das für den Nachweis genetischer Polymorphismen geeignete, auf "adjacent hybridization probes" beruhende Detektionsverfahren hat den zusätzlichen Nachteil, dass bei diesem Verfahren eingesetzte Sonden sehr lange (>40 bp) Hybridisierungsareale benötigen, was deren Anwendung besonders dann erschwert, wenn hypervariable, nur kurze Konservierungsbereiche enthaltende, insbesondere RNA-Targets, detektiert werden sollen. Weitere Nachteile bestehen darin, dass insbesondere das sehr häufig eingesetzte TaqMan-Verfahren wenig robust und sehr störanfällig insbesondere gegenüber inhibierenden Substanzen in der Probe ist. Ein weiterer Nachteil des TaqMan-Verfahrens besteht darin, das es zum Nachweis genetischer Polymorphismen, insbesondere einzelner Mismatche (z.B. Punktmutationen) nur bedingt geeignet ist, da oftmals Einzelbasenaustausche innerhalb der zum Nachweis herangezogenen komplementären Sondensequenzen nicht ausreichen, um Wildtyp-Allele (wt) eindeutig von mutierten Allelen (mt) zu unterscheiden. Die meisten o.g. Detektionsverfahren haben zudem den Nachteil, dass die verwendeten Sonden eine kostenintensive 3'-OH blockierende Modifizierung aufweisen müssen, um eine unerwünschte Elongation der Sonde im PCR-Prozess zu verhindern.

Im Dokument EP-A-1 389 638 wurde ein Verfahren zum Nachweis von Nukleinsäuren beschrieben, wobei an das jeweilige 5'- bzw. 3'-Ende der angrenzenden Hybridisierungssonden eine Stem-ausbildende Struktur angehängt wurde, mit dem Ziel, die an die Stem-Enden angefügten Donor- und Akzeptorfarbstoffe in größere sterische Nähe zueinander zu rücken, um ein höheres Messsignal zu erzielen. Eine Hydrolyse der hybridisierten Sonden wurde darin nicht beschrieben.

Die Druckschrift WO 99/42616 A beschreibt ein Hybridisierungsverfahren, d.h. das Signal wird immer nur durch Hybridisierung der Sonden am Target erzeugt. Es wird niemals eine Sonde hydrolysiert und damit verbraucht. Das bedeutet, dass das dieses Verfahren nur mittels Fluoreszenzmessung funktionieren kann, wenn beide Sonden mit je einem Donor oder Akzeptor markiert sind. Es werden auch keine markierte Fragmente generiert.

WO 01/90399 A offenbart ein Verfahren zur Detektion von Mutationen and Polymorphismen in Nukleinsäuren. Eine Probe enthält dabei ein Polynukleotid, das mit drei Oligonukleotiden kombiniert ist. Eine Stem-Struktur wird darin nicht erwähnt.

Der Erfindung liegt deshalb die Aufgabe zugrunde, die Nachteile der im Stand Technik beschriebenen Lösungen zu beseitigen.

Die Aufgabe wurde durch ein Verfahren zur Detektion von Ziel-Nukleinsäuren oder Nukleinsäureanaloga (Targets) gelöst, bei dem mindestens ein Paar ligand-markierter Oligonukleotide oder Oligonukleotidanaloga (Sonden) oder mindestens ein ligand-markiertes Sondenterzett zum Nachweis genetischer Polymorphismen eingesetzt wird, wobei die upstream Sonde(n) anteilig mit ihrem 5'-Ende an das Target und mit ihrem 3'-Ende anteilig an das 5'-Ende der downstream Sonde hybridisieren und das 3'-Ende der downstream Sonde wiederum anteilig an das Target hybridisiert, so dass im Resultat eine Triplex-Struktur zwischen der Ziel-Nukleinsäure und einem Sondenpaar ausgebildet wird. Eine optimale Hybridisierung der Sonden an das Target erfolgt dabei nur mit dem Sondenpaar, nicht aber mit den Einzelsonden, da die Hybrisisierungsareale einer einzeln verwendeten Sonde zu kurz für eine stabile Hybridisierung zwischen Target-komplementärem Sondenanteil und Target sind. Die anteilig zur Target-Hybridisierung befähigten Sequenzabschnitte der upstream und downstream Sonden hybridisieren vorzugsweise an benachbarte Sequenzen des Targets und gleichzeitig wird zwischen dem 3'-Ende der upstream Sonde und dem 5'-Ende der downstream Sonde eine stabilisierende "Stem-Struktur" ausgebildet. Die upstream Sonde ist mit je einem als Reporter und Quencher fungierenden Liganden markiert und weist keine 3'-OH-Blockierung auf, während die downstream Sonde unmarkiert und am 3'-OH blockiert ist.

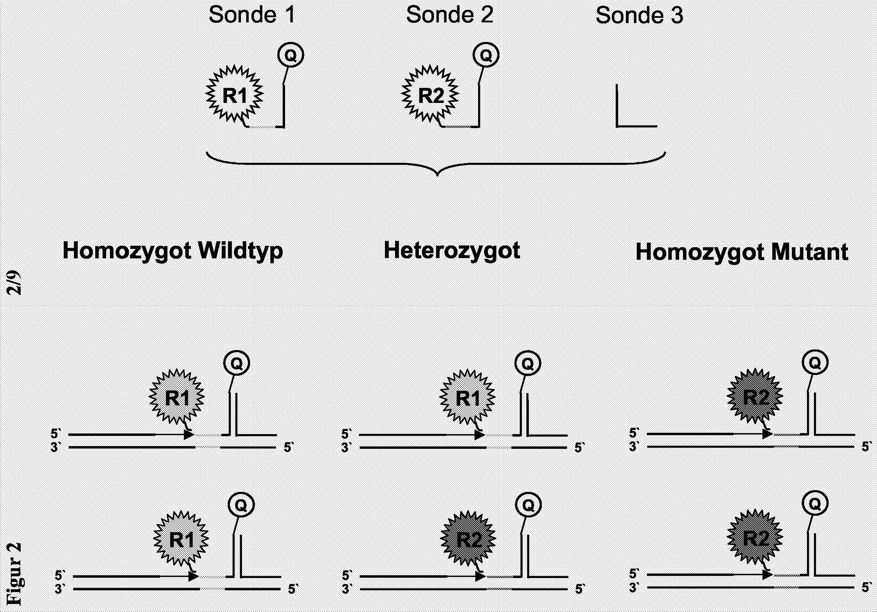

Das erfindungsgemäße Verfahren hat den Vorteil, dass sich sowohl am 5'- als auch am 3'-Ende beider Sonden Markierungen befinden können und so insgesamt 4 verschiedene Loci für verschiedene Kombinationen von Markierungen zur Verfügung stehen. Ein erfindungsgemäßes Sondenterzett zum Nachweis genetischer Polymorphismen (Figur 2) besteht aus 2 markierten upstream oder downstream Sonden, die sich in der Sequenz um mindestens 1 Base unterscheiden und einer zugehörigen upstream oder downstream Sonde, die zur Ausbildung eines Stems mit beiden, dem Sondenterzett angehörenden, upstream oder downstream Sonden befähigt ist, wobei immer nur dann eine hydrolysierbare Triplex-Struktur zwischen upstream Sonde, downstream Sonde und Target gebildet wird, wenn eine ausreichende Komplementarität zwischen dem targethybridisierenden Sondenabschnitt mindestens einer der Sonden und dem Target besteht. Die beiden upstream Sonden sind mit zwei, bei unterschiedlichen Wellenlängen emittierenden, als Reporter fungierenden Liganden sowie einem als Quencher fungierenden Liganden markiert, und weisen keine 3'-OH-Blockierung auf, während die downstream Sonde unmarkiert und am 3'-OH blockiert ist.

Die zwischen dem Sondenpaar und dem Target ausgebildete Triplexstruktur ist entweder direkt infolge des Hybridisierungsereignisses zur Ausbildung eines Mess-Signals befähigt oder es wird erst nach partieller Hydrolyse des Sondenpaares, z.B. durch ein im homogenen Assay gleichfalls enthaltenes Enzym, vorzugsweise eine Polymerase, ein Mess-Signal generiert.

Zum Gegenstand der Erfindung gehört auch mindestens ein Sondenpaar oder ein Sondenterzett zur Detektion von Ziel-Nukleinsäuren (Targets) oder genetischen Polymorphismen, wobei das 5'-Ende der upstream Sonde ausreichend komplementär zu einer Subsequenz des Targets und das 3'-Ende dieser Sonde ausreichend komplementär zum 5'-Ende der downstream Sonde ist und das 3'-Ende der downstream Sonde wiederum ausreichend komplementär zum 5'-Ende der upstream Sonde und das 3'-Ende dieser Sonde wiederum ausreichend komplementär zu einer weiteren Subsequenz des Targets ist.

Überraschenderweise hat sich herausgestellt, dass entweder eine oder beide Sonden - im Unterscheid zum Stand der Technik - keine 3'-OH-Blockierung aufweisen müssen.

Als Sondenpaar oder als Sondenterzett können alle Oligonukleotide oder Oligonukleotidanaloga eingesetzt werden, die in der Lage sind, eine Stem-Struktur auszubilden. Beispielsweise kann die Ausbildung der Stem-Struktur durch die Subsequenzen 5'-AGTCGGAACCTT-3' und 5'-AAGGTTCCGACT-3' oder 5'-AGTCGGAAC-3' und 5'-GTTCCGACT-3' erfolgen. Die Stemstrukturen für das Sondenterzett sind identisch den Stems für ein Sondenpaar, nur dass beispielsweise der Stem 5'-AGTCGGAAC-3' in beiden upstream Sonden enthalten ist, während der Stem 5'-GTTCCGACT-3' nur in der einzig verwendeten downstream Sonde vorliegt..

Erfindungsgemäß tragen eine oder beide Sonden am 5'- und / oder am 3'-Ende eine Markierung. Als Markierung können beispielsweise Fluoreszenz- und/oder Quencherfarbstoffe eingesetzt werden. Es ist auch möglich, eine Sonde am 3'-Ende und die andere Sonde am 5'-Ende mit jeweils einem Donor- und einem Akzeptorfarbstoff zu markieren.

Damit wurde ein universell einsetzbares, robustes Sondenformat entwickelt, das für mehrere auf FRET basierende Nachweisverfahren geeignet ist, den Nachteil der Ausbildung von Duplexstrukturen zur Messung von heteropolymeren Zielsequenzen dadurch abstellt, dass bis zu 2 Messimpulse pro Sonden-Target-Komplex generiert werden können, keine 3'-OH Blockade erforderlich ist sowie der Nachteil des "Adjacent hybridization probes"-Verfahrens dahingehend beseitigt, dass nunmehr kürzere, konservierte Genomabschnitte für die Sondenhybridisierung nutzbar gemacht werden können, was insbesondere den Nachweis sehr variabler Zielsequenzen verbessert.

Das erfindungsgemäße Herstellungsverfahren des Sondenpaares umfasst folgende Schritte:
- Aufspaltung einer bekannten oder unbekannten, dem Target-kompatiblen Oligonukleotid-Sondensequenz von vorzugsweise 15-40 Basen Länge, in zwei Teilsondensequenzen
- Ergänzung der Teilsondensequenzen mit je einer eine Stem-Struktur zwischen den Sonden des Sondenpaares ausbildenden Subsequenze nach Anspruch 8
- chemische Kopplung eines komplexen Liganden, Moleküls oder Atoms, das ein messbares, vorzugsweise quantifizierbares, Signal generieren kann (Markierungen).

Vorzugsweise weisen sowohl die an das Target hybridisierenden Teilsondensequenzen untereinander als auch die Sonden des Sondenpaares jeweils ähnliche Tₘ-Werte auf.

Das erfindungsgemäße Sondenpaar oder Sondenterzett und / oder das Verfahren zur Detektion von Ziel-Nukleinsäuren eignen sich zum qualitativen und / oder quantitativen Nachweis beliebiger heteropolymerer Ziel-Nukleinsäuren oder Nukleinsäureanaloga oder zum Nachweis von beliebigen genetischen Polymorphismen.

### Prinzip der Erfindung

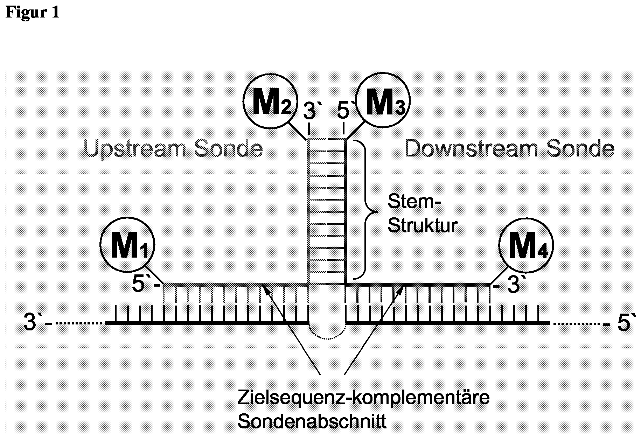

Das Prinzip der Erfindung ist in Figur 1 dargestellt. Ein erfindungsgemäßes Sondenpaar besteht aus zwei Sonden, wobei jede einzelne Sonde immer aus 2 unterschiedlichen funktionellen Sequenz-Abschnitten besteht, einem Zielsequenzkomplementären Sondenabschnitt und einem eine Stem-Struktur zur jeweils zugehörigen zweiten Sonde des Sondenpaares ausbildenden Sondenabschnitt. Unter den gewählten Reaktionsbedingungen ist eine ausreichende Hybridisierung der Sonden nur dem Sondenpaar, nicht aber der Einzelsonde möglich. Experimente mit dem Einsatz von Einzelsonden haben gezeigt, dass diese trotz des verkürzten Hybridisierungsbereiches ans Target binden, auch enzymatisch hydrolysiert werden, aber nur ein deutlich geringeres Fluoreszenzsignal (max. 50 % im Vergleich zum Sondenpaar) in der Sättigungsphase der Reaktion bringen.

Pro Sondenpaar gibt es mindestens 4 mit M1-M4 bezeichnete Loci für die Markierung mit vorzugsweise Fluoreszenz- und/oder Quencherfarbstoffen. In einer bevorzugten Art der Ausgestaltung sind Sonde 1, oder Sonde 2, oder Sonde 1 und Sonde 2 mit je einem oder zwei Reporterfarbstoffen, beispielsweise den Fluorescein- oder Rhodaminfarbstoffen FAM, VIC, JOE, HEX, NED, Yakima Yellow, ROX, Cy3, Cy5 oder DYXL, und einem oder zwei Quencherfarbstoffen wie beispielsweise TAMRA, DABCYL, DABSYL, ElleQuencher oder DarkQuencher markiert. In einer weiteren bevorzugten Art der Ausgestaltung sind beide Sonden vorzugsweise im Stem-Bereich (M2 + M3) mit jeweils einem Donor- und einem Akzeptorfarbstoff wie beispielsweise Fluorescein, LCRed640 oder LCRed705 markiert. In Tabelle 1 sind einige Kombinationsmöglichkeiten für die Markierung dargestellt.

Die Entwicklung der Sondenpaare erfolgt über die Aufspaltung einer bekannten, dem jeweils gewählten Amplifikationssystem-kompatiblen Oligonukleotid-Sondensequenz, beispielsweise einer TaqMan-Sonde, in zwei Teilsondensequenzen mit vorzugsweise ähnlichem Tₘ-Wert und Ergänzung der Teilsondensequenzen um geeignete komplementäre, eine spätere Stem-Struktur ausbildende Teilsequenzen. Die Herstellung der Sondenpaare erfolgt mittels dem einem Fachmann bekannten Verfahren der konventionellen Oligonukleotid-Festphasensynthese und anschließender chemischer Kopplung der vorzugsweise verwendeten chromophoren Liganden.

Tabelle 1: Beispiele für verschiedene Kombinationsmöglichkeiten von Markierungen für das erfindungsgemäße Sondenpaar (Figur 1). Bezeichnungen: Reporter (R), Quencher (Q), Donor (D), Akzeptor (A), keine Markierung (Ø)

| Variante | M1 | M2 | M3 | M4 |
|---|---|---|---|---|
| 1 | R | Q | Ø | Ø |
| 2 | Ø | Ø | R | Q |
| 3 | R | Q | R | Q |
| 4 | Ø | D | A | Ø |
| 5 | R | Ø | Q | Ø |
| 6 | R | Q | Ø | Q |
| 7 | Ø | Q | R | Q |

Überraschenderweise zeigte sich beim Einsatz des Sondenpaares, dass im Gegensatz zu anderen Sondenformaten des Standes der Technik weder für Sonde 1 noch für Sonde 2 des erfindungsgemäßen Sondenpaares eine 3'-OH Blockade erforderlich ist.

Eine mögliche Form der Anwendung ist der Einsatz der erfmdungsgemäßen Sondenpaare in dem Fachmann bekannten enzymatischen Amplifizierungsreaktionen, beispielsweise der Polymerasekettenreaktion (PCR).

Das Wesen der Erfindung liegt somit in einer Kombination bekannter Elemente und neuer Lösungswege, die sich gegenseitig beeinflussen und in ihrer neuen Gesamtwirkung einen Gebrauchsvorteil und den erstrebten Erfolg ergeben, der darin liegt, dass nunmehr eine Triplex-Struktur mit der Zielsequenz ausbildende Sondenpaare für den sensitiven homogenen Nachweis beliebiger ausgewählter Nukleinsäuren oder den Nachweis beliebiger genetischer Polymorphismen in biologischen Substanzen zur Verfügung stehen.

Die Anwendung der erfindungsgemäßen Sondenpaare besteht im Einsatz für die validierte, standardisierte Detektion von Zielsequenz DNA oder RNA in vorzugsweise biologischen Untersuchungsmaterialien, wobei einzelne Oligonukleotide, insbesondere Sonden und Primer, auch aus dem System herausgelöst separat für PCR-unabhängige enzymatische Amplifizierungsreaktionen, gegebenenfalls auch in Verbindung mit anderen Detektionstechniken nutzbar sein können.

### Figuren:

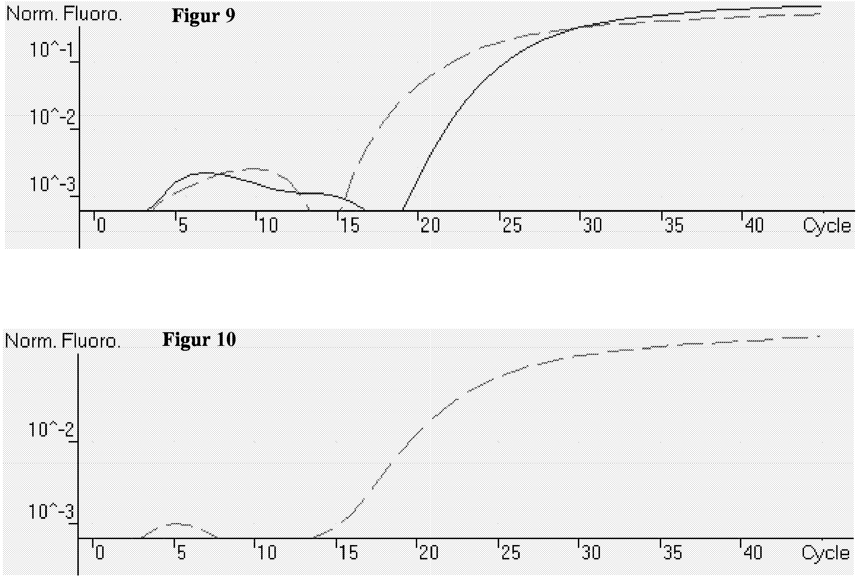

**Figur 1****:** Funktionsprinzip des erfindungsgemäßen Sondenpaares
**Figur 2****:** Funktionsprinzip des erfindungsgemäßen Sondenterzetts zum Nachweis genetischer Polymorphismen.
   Das Sondenterzett besteht immer aus einer wildtyp-spezifischen Sonde und einer mutationsspezifischen Sonde, die sondenspezifisch mit 2 unterscheidbaren ReporterFarbstoffen (R1 und R2) und einem Quencher-Farbstoff (Q) markiert sind sowie einer sowohl mit der wildtyp- als auch der mutationsspezifischen Sonde eine Stem-Struktur ausbildenden, unmarkierten Sonde. Reine Signale der R1- oder R2-Sonden sind immer nur dann nachweisbar, wenn das nachzuweisende Allel entweder homozygot als Wildtyp oder Mutant in der Probe vorliegt. Sind hingegen R1- und R2-Signale gleichzeitig messbar, so spricht dies für eine heterozygote Allelkombination, d.h. das Vorhandensein je eines Wildtyp- und eines mutanten Allels in der Probe.

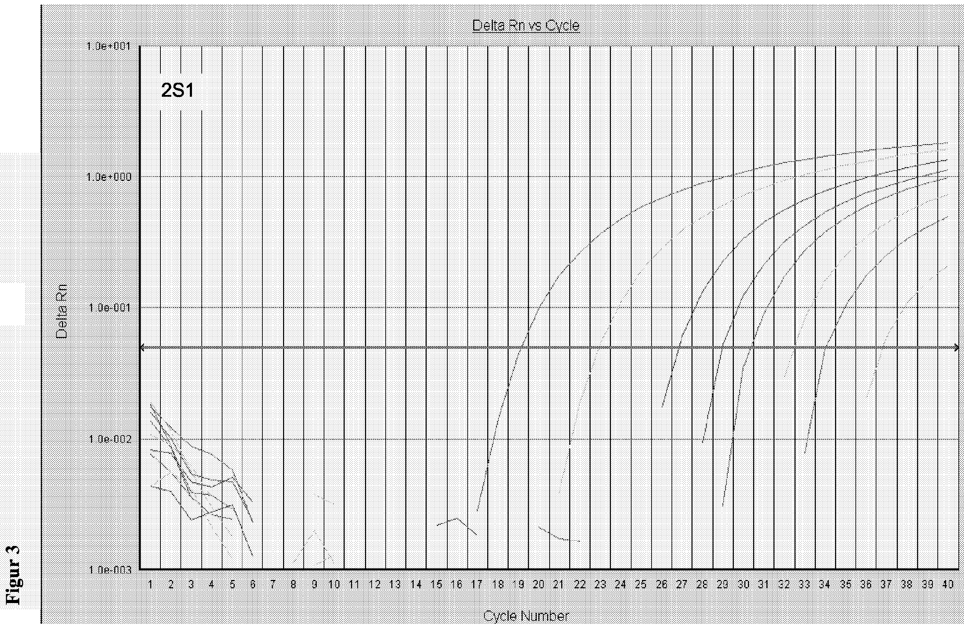

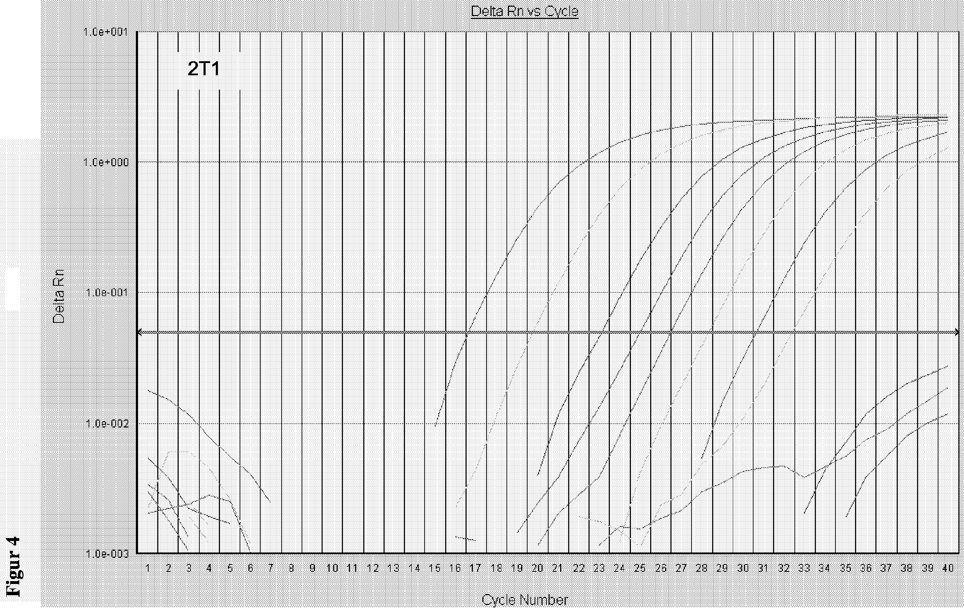

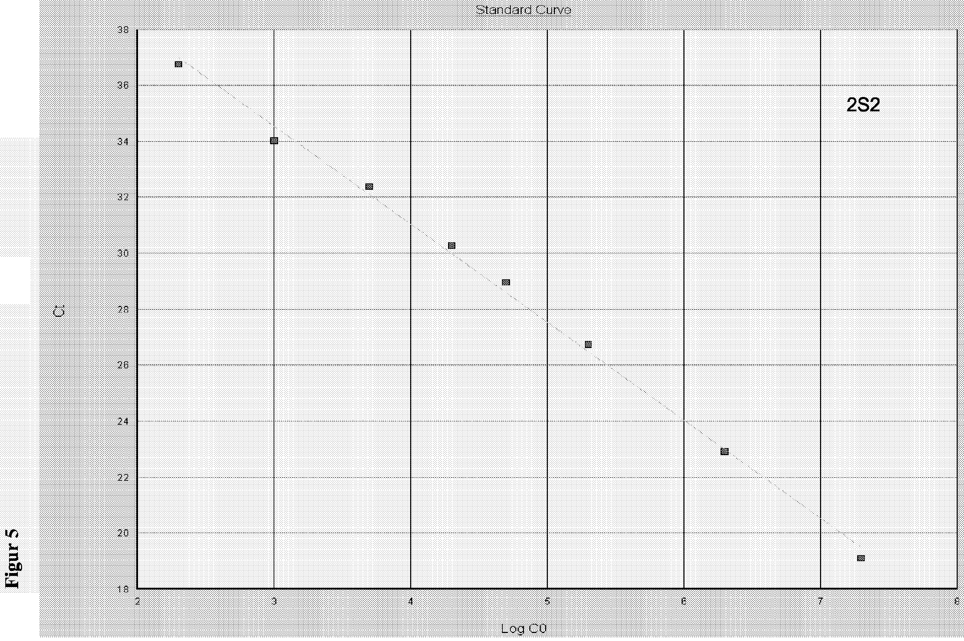

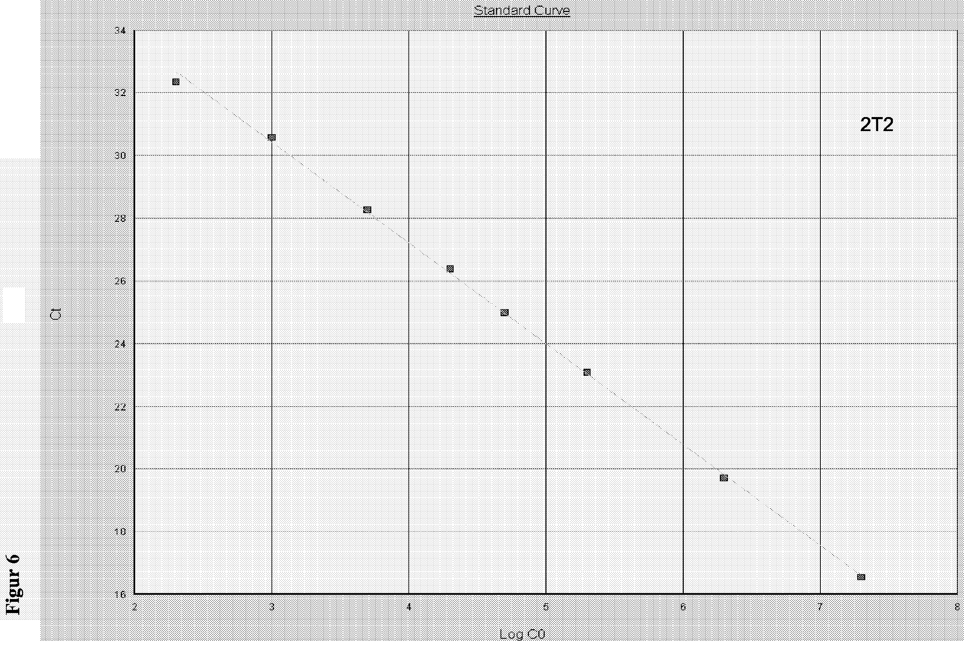

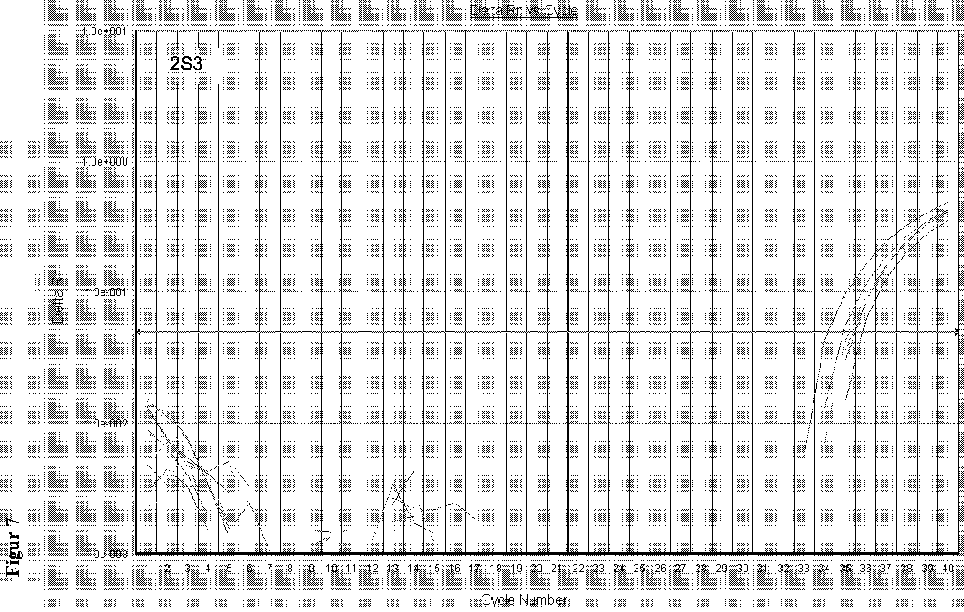

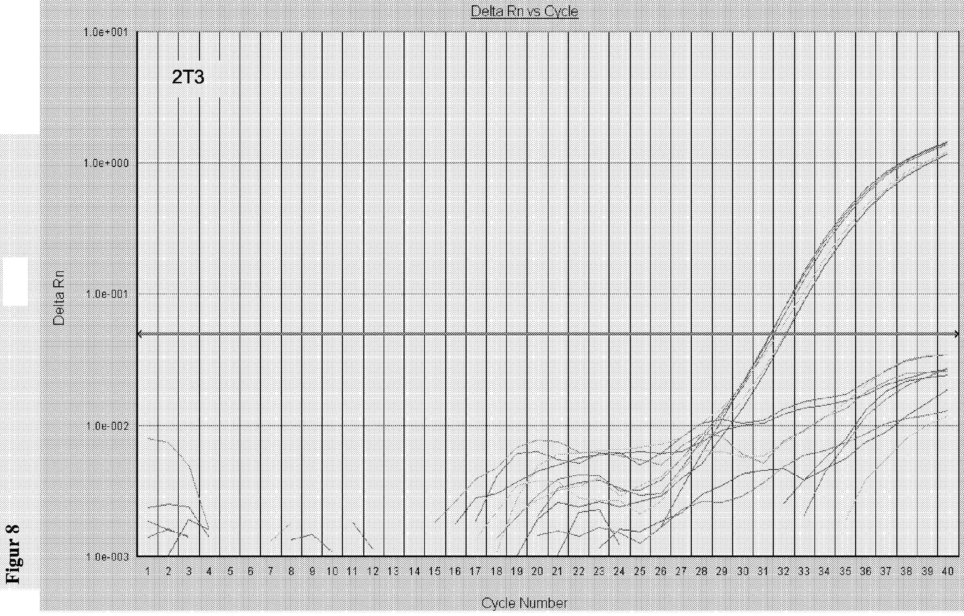

**Figuren 3 bis 8****:** Einsatz eines erfindungsgemäßen Sondenpaares zur Messung von Hepatitis B Viren (HBV) über Sequenzabschnitte des Surface antigen Gens mittels quantitativer PCR (2S). Vergleich der Validität mit einem ähnlichen quantitativen TaqMan-Protokoll (2T) unter Verwendung identischer Zielsequenzen.
   **2S1:** Real-time PCR Amplifikation einer HBV Standard-DNA unter Nutzung des erfindungsgemäßen Sondenpaars, Sättigungskurven
   **2S2:** Real-time PCR Amplifikation einer HBV Standard-DNA unter Nutzung des erfindungsgemäßen Sondenpaars, Standard-Referenzkurve, Slope = -3,498; Intercept = 45,028, R2 = 0,997
   **2S3:** Real-time PCR Amplifikation einer klinischen HBV-Probe, aus der zuvor die DNA gereinigt wurde, unter Nutzung des erfindungsgemäßen Sondenpaars, Sättigungskurven
   **2T1:** Real-time PCR Amplifikation einer HBV Standard-DNA unter Nutzung eines vergleichbaren TaqMan-Protokolls, Sättigungskurven
   **2T2:** Real-time PCR Amplifikation einer HBV Standard-DNA unter Nutzung eines vergleichbaren TaqMan-Protokolls, Standard-Referenzkurve, Slope = -3,209; Intercept = 40,046, R2 = 0,999
   **2T3:** Real-time PCR Amplifikation einer klinischen HBV-Probe, aus der zuvor die DNA gereinigt wurde, unter Nutzung eines vergleichbaren TaqMan-Protokolls, Sättigungskurven
**Figuren 9** **bis 10:** Diagnostik der Faktor V Leiden (FV) Mutation in genomischer DNA.

Zwei für die Mutation 1691 G→A prä-typisierte DNA-Probandenproben (eine heterozygote, eine homozygote wt-Probe) wurden unter Einsatz des erfindungsgemäßen Verfahrens nach Ausführungsbeispiel 4 typisiert. Die heterozygote DNA-Pobandenprobe (Probe 1) erbrachte erwartungsgemäß sowohl im FAM (Figur 9) als auch im JOE (YY)-Kanal (Figur 10) eine sigmoide Sättigungskurve (gestrichelte Kurve). Dies beweist das Vorliegen einer heterozygoten Allelkombination in der Probe 1, d.h. das Vorhandensein je eines Wildtyp- und mutierten Allels. Die Messung eines sigmoiden Fluoreszenzsignals ausschließlich im FAM-Kanal für Probe 2 beweist hingegen die Existenz einer Wildtyp-Allelkombination in der Probe 2, d.h. das Vorhandensein von zwei Wildtyp-Allelen.

### Ausführungsbeispiele

### Beispiel 1: Erfindungsgemäßes Sondenpaar zur Messung von Hepatitis B Viren (HBV) über Sequenzabschnitte des Surface antigens, HBsAg Gene mittels quantitativer PCR

**Verwendete Oligonukleotide (GeneBank Accession M20919):**

| | | |
|---|---|---|
| SEQ ID No. 1 | Primer1_HBV [233] | 5' 520-5413' |
| | | 5'-TGTCCTCCAATTTGTCCTGGTT-3' |
| | | |
| SEQ ID No. 2 | Primer2_HBV [234] | 5' 570-592 3' |
| | | 5'-GCAGCAGGATGAAGAGGAATATG-3' |
| | | |
| SEQ ID No. 3 | Sonde 1 [393] | 5' 544-554 3' |
| | | 5'-FAM-CGCTGGATGTGAGTCGGAACCTT-3'-BHQ1 |
| | | Tₘ = 79,1 °C |
| | | |
| SEQ ID No. 3.1 | Sonde 1 [400] | 5' 544-554 3' |
| | | 5'-FAM-CGCTGGATGTGAGTCGGAAC-3'-BHQ1 |
| | | Tₘ = 79,1 °C |
| | | |
| SEQ ID No. 4 | Sonde 2 [394] | 5' 555-563 3' |
| | | 5'-AAGGTTCCGACTTCTGCGGCG-3' |
| | | Tₘ = 79,2 °C |
| | | |
| SEQ ID No. 4.1 | Sonde 2 [413] | 5' 555-563 3' |
| | | 5'-GTTCCGACTTCTGCGGCG-3'-OH-P |
| | | Tₘ = 79,2 °C |

Die Tₘ-Werte wurden mittels Oligo Primer Analysis Software vers. 6.23 (Molecular Biology Insights, Inc.) nach der %GC-Regel berechnet. Die unterstrichenen Sequenzen stellen die jeweils komplementären, Stem-ausbildenden Abschnitte des erfindungsgemäßen Sondenpaares dar.

### Amplifikation:

Die mittels SEQ ID No. 1 und SEQ ID No. 2 amplifizierte Region des HBsAg Gens resultierte in einem Amplikon von 73 bp Länge. Für den real-time PCR Einsatz des erfindungsgemäßen Sondenpaares am ABI PRISM 7000 Sequence Detection System wurde folgendes optimiertes Protokoll für einem 25-µl PCR-Ansatz gewählt (Tabelle 2):

**Tabelle 2: Verwendete Reagenzien und optimierte Konzentrationen für den Einsatz das erfindungsgemäßen Sondenpaares**

| | **25 µl**-**Ansätze** | | |
|---|---|---|---|
| **Reaktionskomponenten im Ansatz** | **Volumen pro Ansatz (µl)** | **Volumen Mastermix (µl)** | **AnsatzKonzentration** |
| H₂O (PCR grade) | 10,7 | 128,4 | - |
| 10x ROX-Puffer | 2,5 | 30,0 | 1x |
| MgCl₂ (50 mM) | 3,5 | 42,0 | 7 mM |
| Nukleotid-Mix (2,5 mM dATP, 2,5 mM dCTP, 2,5 mM dGTP, 5 mM dUTP) | 2,0 | 24,0 | 0,2/ 0,4 mM (dUTP) |
| SEQ ID No. 1 (15 µM) | 0,5 | 6,0 | 300 nM |
| SEQ ID No. 2 (15 µM) | 0,5 | 6,0 | 300 nM |
| SEQ ID No. 3 (2,0 µM) | 2,5 | 30,0 | 200 nM |
| SEQ ID No. 4 (2,0 µM) | 2,5 | 30,0 | 200 nM |
| HBV Standard-DNA (HBV-DNA beschichteter 8 Tubes/Strip) | - | - | Siehe Tabelle 3 |
| AmpliTaq Gold DNA Polymerase (5 U/µl, Applied Biosystems) | 0,3 | 3,6 | 1,5 |

**Tabelle 3: Konzentrationen der verwendeten HBV-Standards, kalibriert gegen WHO-Referenzmaterial und bezogen auf 1 ml Untersuchungsmaterial (Serum oder Plasma).**

| **Tube Nr.** | **DBV Standard-DNA [IU/ml]** | **HBV Standard**-**DNA [Kopien/ml]** |
|---|---|---|
| 1 | 20,000,000 | 100,000,000 |
| 2 | 2,000,000 | 10,000,000 |
| 3 | 200,000 | 1,000,000 |
| 4 | 50,000 | 250,000 |
| 5 | 20,000 | 100,000 |
| 6 | 5,000 | 25,000 |
| 7 | 1,000 | 5,000 |
| 8 | 200 | 1,000 |

Die Reagenzien aus Tabelle 2 wurden initial zu einem Mastermix ausreichend für 12 PCR-Ansätze zusammengeführt. 25-µl-Aliquote des Mastermixes wurden in die 8 mit verschiedenen Konzentrationen von Standard HBV-DNA beschichteten Reaktionsgefäße (Roboscreen, DE 198 40 531) sowie 3 nicht mit Standard HBV-DNA beschichtete Reaktionsgefäße (PCR-Leerwerte) pipettiert. Die Konzentrationen der verwendeten Standards sind in Tabelle 3 dargestellt. Amplifikation und Detektion der PCR-Produkte erfolgten real-time unter Nutzung eines ABI PRISM 7000 Sequence Detection Systems (Applied Biosystems). Folgendes Thermoprofil wurde gewählt:

| | | | |
|---|---|---|---|
| Initiale Denaturierung | | 95°C | 10:00 min |
| 40 Zyklen (3-Step-PCR) | Denaturierung | 95°C | 00:30 min |
| | Annealing | 45°C | 00:15 min |
| | Extension | 57°C | 01:15 min |

### Ergebnis:

Während die PCR-Leerwerte keinerlei exponentielles Amplifikationsignal erbrachten, d.h. keine Kontamination des Nachweissystems vorhanden war, war in allen Standard HBV-DNA beschichteten Reaktionsgefäßen ein gewünschtes Amplifikationssignal in Form einer Sättigungskurve nachweisbar (Figur 2S1). Die Auswertung der Kurven ermöglichte die Erstellung einer Standard-Referenzkurve (Figur 2S2) welche bezüglich den Auswertungsparametern Anstieg (Slope), Ordinatendurchgang (Intercept), und linearer Regression (R2) den dem Fachmann bekannten Erwartungswerten entspricht. Die Daten sind qualitativ vergleichbar zum im Beispiel 2 angewandten, etablierten TaqMan-Verfahren (Beispiel 2, Figur 2T1 und 2T2).

### Beispiel 2: TaqMan-Methode zur Messung von Hepatitis B Viren (HBV) über Sequenzabschnitte des Surface antigens, HBsAg Gene mittels quantitativer PCR

Zusätzlich zu SEQ ID No. 1 und SEQ ID No. 2 (Beispiel 1) verwendete Oligonukleotide (GeneBank Accession M20919):

| | | |
|---|---|---|
| SEQ ID No. 5 | HBV_Prob [235] | 5' 544-563 3' |
| | | 5'-FAM-CGCTGGATGTGTCTGCGGCG-3'-TAMRA |
| | | Tₘ = 81,4 °C |

Die Tₘ-Werte wurden mittels Oligo Primer Analysis Software vers. 6.23 (Molecular Biology Insights, Inc.) nach der %GC-Regel berechnet.

### Amplifikation:

Die mittels SEQ ID No. 1 und SEQ ID No. 2 amplifizierte Region des HBsAg Gens resultierte in einem Amplikon von 73 bp Länge. Für den real-time Nachweis des Amplikons mittels SEQ ID No. 5 unter Nutzung eines ABI PRISM 7000 Sequence Detection Systems wurde folgendes optimiertes Protokoll für einem 25-µl PCR-Ansatz gewählt (Tabelle 4):

Die Reagenzien aus Tabelle 4 wurden initial zu einem Mastermix ausreichend für 12 PCR-Ansätze zusammengeführt. 25-µl-Aliquote des Mastermixes wurden in die 8 mit verschiedenen Konzentrationen von Standard HBV-DNA beschichteten Reaktionsgefäße (Roboscreen, DE 198 40 531) sowie 3 nicht mit Standard HBV-DNA beschichtete Reaktionsgefäße (PCR-Leerwerte) pipettiert. Die Konzentrationen der verwendeten Standards sind in Tabelle 3 dargestellt. Amplifikation und Detektion der PCR-Produkte erfolgten real-time unter Nutzung eines ABI PRISM 7000 Sequence Detection Systems (Applied Biosystems). Folgendes Thermoprofil wurde gewählt:

**Tabelle 4. Verwendete Reagenzien und optimierte Konzentrationen für das TaqMan-Protokoll**

| | **25 µl**-**Ansätze** | | |
|---|---|---|---|
| **Reaktionskomponenten im Ansatz** | **Volumen pro Ansatz (µl)** | **Volumen Mastermix (µl)** | **Ansatz-Konzentration** |
| H₂O (PCR grade) | 16,2 | 194,4 | - |
| 10x ROX-Puffer | 2,5 | 30,0 | 1x |
| MgCl₂ (50 mM) | 1,5 | 18,0 | 3 mM |
| Nukleotid-Mix (2,5 mM dATP, 2,5 mM dCTP, 2,5 mM dGTP, 5 mM dUTP) | 2 | 24,0 | 0,2/ 0,4 mM (dUTP) |
| SEQ ID No. 1 (15 µM) | 0,5 | 6,0 | 300 nM |
| SEQ ID No. 2 (15 µM) | 0,5 | 6,0 | 300 nM |
| SEQ ID No. 5 (2,0 µM) | 1,5 | 18,0 | 120 nM |
| HBV Standard-DNA (HBV-DNA beschichteter 8 Tubes/Strip) | - | - | Siehe Tabelle 3 |
| AmpliTaq Gold DNA Polymerase (5 U/µl, Applied Biosystems) | 0,3 | 3,6 | 1,5 |

| | | | |
|---|---|---|---|
| Initiale Denaturierung | | 95°C | 10:00 min |
| 40 Zyklen (2-Step-PCR) | Denaturierung | 95°C | 00:15 min |
| | Annealing/Extension | 59°C | 01:15 min |

### Ergebnis:

Während die PCR-Leerwerte keinerlei exponentielles Amplifikationsignal erbrachten, d.h. keine Kontamination des Nachweissystems vorhanden war, war in allen Standard HBV-DNA beschichteten Reaktionsgefäßen ein gewünschtes Amplifikationssignal in Form einer Sättigungskurve nachweisbar (Figur 2T1). Die Auswertung der Kurven ermöglichte die Erstellung einer Standard-Referenzkurve (Figur 2T2), welche bezüglich den Auswertungsparametern Anstieg (Slope), Ordinatendurchgang (Intercept), und linearer Regression (R2) den dem Fachmann bekannten Erwartungswerten entspricht.

### Beispiel 3 Praxis-Vergleich des erfindungs gemäßen Verfahrens mit dem etablierten TaqMan-Verfahren anhand HBV-Viren enthaltender, klinischer Proben

### Klinische Probe:

Als klinische HBV-positive Probe wurde HBV Referenzplasma des Paul-Ehrlich-Instituts (50,000 IU/ml (Lot #1872/01, genotype D, subtype ayw2/3) verwendet. Die Probe wurde mit *HBV* negativem Plasma eines HBV-gesunden Blutspenders auf 1000 IU/ml verdünnt. Als Negativkontrolle diente das *HBV* negative Plasma.

### Nukleinsäurereinigung aus der klinischen Probe:

Zur Reinigung der DNA aus der HBV-positive Probe und der Negativkontrolle wurde ein RTP^{®} DNA/RNA Virus *Mini* Kit (Roboscreen GmbH) nach den Angaben des Herstellers eingesetzt. Die Extraktion erfolgte in Dreifach-Ansätzen aus jeweils 200 µl des HBV-Positivplasmas und des HBV-Negativplasmas. Die Elution der gereinigten DNA erfolgte mit je 60 µl des im Kit enthaltenen Elutionspuffers.

### Amplifikation:

Die mittels SEQ ID No. 1 und SEQ ID No. 2 amplifizierte Region des HBsAg Gens resultierte in einem Amplikon von 73 bp Länge. Für die "real-time" Quantifzierung der HBV-Kopien mittels des erfindungsgemäßen Verfahrenes und dem TaqMan-Kontrollverfahren wurden die im Beispiel 1 und 2 ausgeführten Protokolle verwendet. Die Mastermixe wurden wie in Tabelle 5 gezeigt hergestellt.

Die Reagenzien in Tabelle 5 wurden initial zu einem Mastermix ausreichend für 13 PCR-Ansätze zusammengeführt und in 20-µl-Aliquoten in die entsprechende Anzahl (je 12) von 0,2 ml DNA Sample-Reaktionsgefäße (Roboscreen) verbracht. Die HBV-positiven und HBV-negativen klinischen Proben wurden mit beiden verglichenen Verfahren je in Doppelbestimmung amplifiziert, d.h. es wurden je 6 identische PCR-Ansätze pro Verfahren unter Nutzung einer identischen Probe (1000 IU/ml) hergestellt. Amplifikation und Detektion der PCR-Produkte erfolgten real-time unter Nutzung eines ABI PRISM 7000 Sequence Detection Systems (Applied Biosystems). Die Thermoprofile wurden wie in Beispiel 1 (Sondenpaar) und Beispiel 2 (TaqMan) gewählt.

**Tabelle 5: Verwendete Reagenzien und Herstellung der Mastermixe**

| | **Sondenpaar** | | **TaqMan** | |
|---|---|---|---|---|
| **Reaktionskomponenten im Ansatz** | **Vol. pro Ansatz (µl)** | **Mastermix (µl)** | **Vol. pro Ansatz (µl)** | **Mastermix (µl)** |
| H₂O (PCR grade) | 7,7 | 100,1 | 11,2 | 145,6 |
| 10x ROX-Puffer | 2,5 | 32,5 | 2,5 | 32,5 |
| MgCl₂ (50 mM) | 1,5 | 19,5 | 1,5 | 19,5 |
| Nukleotid-Mix (2,5 mM dATP, 2,5 mM dCTP, 2,5 mM dGTP, 5 mM dUTP) | 2,0 | 26,0 | 2,0 | 26,0 |
| SEQ ID No. 1 (15 µM) | 0,5 | 6,5 | 0,5 | 6,5 |
| SEQ ID No. 2 (15 µM) | 0,5 | 6,5 | 0,5 | 6,5 |
| SEQ ID No. 3 (2,0 µM) | 2,5 | 32,5 | - | - |
| SEQ ID No. 4 (2,0 µM) | 2,5 | 32,5 | - | - |
| SEQ ID No. 5 (2,0 µM) | - | - | 1,5 | 19,5 |
| Gereinigte HBV Proben-DNA | 5 | - | 5 | - |
| AmpliTaq Gold DNA Polymerase (5 U/µl) | 0,3 | 3,9 | 0,3 | 3,9 |

### Ergebnisse:

Während die Negativkontrollen und der PCR-Leerwert (5 µl PCR grade H₂O anstatt gereinigter HBV-DNA Probe im PCR-Ansatz) keinerlei exponentielles Amplifikationsignal erbrachten, d.h. keinerlei Kontamination des Nachweissystems vorhanden war, waren alle HBV-positiven Proben sowohl mittels des erfindungsgemäßen Verfahrens (Figur 2S3) und des TaqMan-Vergleichsverfahrens deutlich amplifizierbar (Figur 2T3). Die quantitative Analyse (Tabelle 6) zeigte, dass der Nachweis von HBV-Kopien mit dem erfindungsgemäßen Sondenpaar im Vergleich zum TaqMan-Verfahren signifikant um ca. 33% effizienter erfolgte (durchschnittlich 744 von 1000 IU/ml gegenüber 417 von 1000 IU/ml). Die Ursache hierfür könnte in einer höheren Robustheit des Verfahrens gegenüber dem als störanfällig bekannten TaqMan-Verfahren liegen.

**Tabelle 6: Mit beiden Verfahren erzielte, quantitative Messergebnisse**

| Messung-Nr. | IU/ml TaqMan | IU/ml Sondenpaar |
|---|---|---|
| 1 | 523 | 1316 |
| 2 | 527 | 673 |
| 3 | 300 | 455 |
| 4 | 466 | 585 |
| 5 | 394 | 822 |
| 6 | 291 | 613 |
| Mittelwert | 417 | 744 |
| Standardabweichung (SD) | 106 | 305 |

### Beispiel 4: Detektion des Faktor V Leiden Polymorphismus (1691 G→A) auf dem Antisense-Strang mit dem erfindungsgemäßen Verfahren

**Verwendete Oligonukleotide (GeneBank Accession Z99572):**

| | |
|---|---|
| SEQ ID No. 6 | FVPrimer1f [425] 5' 62865-62902 3' |
| | 5'-GAAATTCTCAGAATTTCTGAAAGGTTAC-3' |
| | |
| SEQ ID No. 7 | FVPrimer2r [426] 5' 62956-62983 3' |
| | 5'-CCTCTGGGCTAATAGGACTACTTCTAAT-3' |
| | |
| SEQ ID No. 8 | FVmt_S1[427] 5' 62927-62939 3' |
| | 5-YY-CTT*GCCTGTCCAGAGTCGGAAC-3-BHQ1 (ohne 3'-OH Blockade) |
| | Tₘ = 79,2 °C |
| | |
| SEQ ID No. 9 | FVwt_S1 [428] 5' 62927-62939 3' (wildtyp-spezifische Sonde) 5'-FAM-CTC*GCCTGTCCAGAGTCGGAAC-3'-BHQ1 (keine 3'-OH Blockade) |
| | Tₘ = 81,0 °C |
| SEQ ID No. 10 | FV_S2 [429] 5' 62940-62953 3' (mutations-spezifische Sonde) |
| | 5'-GTTCCGACTGGATCTGCTCTTAC-3' (3'-OH Blockade) |
| | Tₘ = 77,3 °C |

| | |
|---|---|
| *mt/wt selektiver Basenaustausch in der Sondensequenz | |

### Klinische Probe:

Als klinische Probandenproben wurde eine synthetische DNA mit typisiertem heterozygoten Genotyp der Faktor V Leiden Mutation (G→A) aus einem zertifizierten Thrombo-Check Kit (DiaTech s.r.l., Italien) (Probe 1) sowie die gereinigte DNA eines als wt-Allelträger prä-typisierten Probanden (Probe 2) verwendet.

### Nukleinsäurereinigung aus der klinischen Probe:

Die chemische Nukleinsäureextraktion aus der Probandenprobe erfolgte mittels DNAzol-Reagenz nach Chromczynski (Chomczynski P. et al., BioTechniques 1997; 22: 550-553.). Von der synthetischen DNA und der 1:10 mit PCR-reinem H₂O verdünnten genomischen DNA aus der Probandenprobe wurde je 2 µl pro PCR-Reaktion eingesetzt. Die Reaktionen wurden in Doppelbestimmung pipettiert.

### Amplifikation:

Die mittels SEQ ID No. 6 und SEQ ID No. 7 amplifizierte Region des humanen Gerinnungsfaktor V Gens resultierte in einem Amplikon von 118 bp Länge. Für die "real-time" Genotypisierung der Faktor V Leiden Mutation 1691 G→A auf dem DNA-Gegenstrang (d.h. C→T Austausch) mittels des erfindungsgemäßen Verfahrenes wurde das nachfolgende Protokoll verwendet. Die Mastermixe wurden wie in Tabelle 7 gezeigt hergestellt.

**Tabelle 7: Verwendete Reagenzien und Herstellung der Mastermixe**

| | **25 µl**-**Ansätze** | | |
|---|---|---|---|
| **Reaktionskomponenten im Ansatz** | **Volumen pro Ansatz (µl)** | **Volumen Mastermix (µl)** | **Ansatz-Konzentration** |
| H₂O (PCR grade) | 13,7 | 68,5 | - |
| 10x Puffer | 2,5 | 12,5 | 1x |
| MgCl₂ (50 mM) | 2,5 | 12,5 | 5 mM |
| Nukleotid-Mix (2,5 mM dATP, 2,5 mM dCTP, 2,5 mM dGTP, 5 mM dUTP) | 2 | 10,0 | 0,2/ 0,4 mM (dUTP) |
| SEQ ID No. 6 (15 µM) | 0,5 | 2,5 | 300 nM |
| SEQ ID No. 7 (15 µM) | 0,5 | 2,5 | 300 nM |
| SEQ ID No. 8 (10,0 µM) | 0,5 | 2,5 | 200 nM |
| SEQ ID No. 9 (10,0 µM) | 0,5 | 2,5 | 200 nM |
| SEQ ID No. 10 (10,0 µM) | 2,0 | 10,0 | 800 nM |
| DNA-Probe | - | - | Siehe unter "klinische Probe" |
| AmpliTaq Gold DNA Polymerase (5 U/µl, Applied Biosystems) | 0,3 | 1,5 | 1,5 |

Die Reagenzien aus Tabelle 7 wurden initial zu einem Mastermix ausreichend für 5 PCR-Ansätze zusammengeführt. 25-µl-Aliquote des Mastermixes wurden in die entsprechenden Reaktionsgefäße pipettiert. Amplifikation und Detektion der PCR-Produkte erfolgten "real-time" unter Nutzung eines Rotor-Gene 3000 "real-time" PCR-Gerätes (Corbett Research). Folgendes Thermoprofil wurde gewählt:

| | | |
|---|---|---|
| Initiale Denaturierung | | 95°C 10:00 min |
| 45 Zyklen (3-Step-PCR) | Denaturierung | 95°C 00:15 min |
| | Annealing | 45°C 00:01 min |
| | Extension | 59°C 00:40 min |

### Ergebnisse:

Figuren 9 und 10 ist zu entnehmen, dass das erfindungsgemäße Verfahren zur Diagnostik der Faktor V Leiden Mutation 1691 G→A in genomischer DNA geeignet ist. Die hinsichtlich Mutation 1691 G→A mittels eines zugelassenen Testkits prä-typisierte DNA-Probandenproben (eine heterozygote, eine homozygote wt-Probe) wurden zweifelsfrei unter Einsatz des erfindungsgemäßen Verfahrens übereinstimmend typisiert. Die Messung eines exponentiellen Fluoreszenzsignals sowohl im FAM (Figur 9) als auch im YY-Kanal (Figur 10) beweist das Vorliegen einer heterozygoten Allelkombination in der Probe 1, d.h. das Vorhandensein je eines Wildtyp- und mutierten Allels. Die Messung eines Fluoreszenzsignals ausschließlich im FAM-Kanal (Figur 9) beweist die Existenz einer Wildtyp-Allelkombination in der Probe 2, d.h. das Vorhandensein von zwei Wildtyp-Allelen.

Das Ergebnis zeigt weiterhin, dass bereits ein Mismatch an prominenter Position in der upstream Sonde (z.B. ein T→C Austausch an Position 3 des 5'-Ende der Sonde) eine Selektivität zwischen Wildtyp- und mutiertem Allel erlaubt.

### Definitionen

### Markierte Oligonukleotide:

Unter markierten Oligonukleotiden versteht der Fachmann Nukleinsäuren oder Nukleinsäureanaloga, die eine oder mehrere, für die Ausführung der Erfindung geeignete Markierung tragen. Unter einer Markierung versteht man jedes Molekül oder Atom, das ein messbares, vorzugsweise quantifizierbares, Signal generieren kann. Markierungen können per Definition in der Lage sein, Mess-Signale entweder in Form von Fluoreszenz, Radioaktivität, Kolorimetrie, Gravimetrie, Röntgenstrahlbeugung oder -absorption, Magnetismus, enzymatischer Aktivität oder Vergleichbarem zu erzeugen. Markierungen inklusive der für die Markierungsreaktion erforderlichen Methoden umfassen, ohne darauf beschränkt zu sein, Enzymsubstrate, radioaktive Atome, Fluoreszenzfarbstoffe, Chromophoren, chemilumineszente Verbindungen, elektrochemilumineszente Verbindungen, Liganden mit spezifischen Bindungspartnern oder jede andere Art von Markierung, die mit weiteren Markierungsliganden interagieren kann, um somit eine Verstärkung, Verringerung oder Veränderung eines Mess-Signals zu bewirken. Vorzugsweise werden solche Markierungen verwendet, die auch bei hohen Temperaturen wie sie beispielsweise bei der PCR Verwendung finden, ihre Stabilität behalten.

### Ziel-Nukleinsäuren (Target)

Der Begriff "Ziel-Nukleinsäure", "Target" oder "Zielsequenz" bezieht sich auf einen Sequenzabschnitt einer heteropolymeren Nukleinsäure oder eines Nukleinsäureanalogon, die entweder amplifiziert, detektiert oder amplifiziert und detektiert werden soll.

### Upstream Sonde:

Die upstream Sonde des Sondenpaares ist in diesem Kontext eine heteropolymere Nukleinsäure oder ein Nukleinsäureanalogon mit einer teilweisen Komplementarität zu einer Subsequenz des Targets, die näher zum 3'-Ende des Targets liegt.

### Downstream Sonde:

Die downstream Sonde des Sondenpaares ist in diesem Kontext eine heteropolymere Nukleinsäure oder ein Nukleinsäureanalogon mit einer teilweisen Komplementarität zu einer Subsequenz des Targets, die näher zum 5'-Ende des Targets liegt.

### Triplex-Struktur:

Eine Triplex-Struktur im Sinne der Erfindung ist eine Komplex bestehend aus 2 heteropolymeren, einzelsträngigen Oligonukleotid-Sonden oder Oligonukleotid-analogen Sonden sowie eines einzelsträngigen Targets, wobei es zu Hybridisierungen, d.h. der Ausbildung von Wasserstoffbrückenbindungen zwischen komplementären Basen sowohl zwischen den Sonden und dem Target als auch zwischen den Sonden untereinander kommt.

### Stem-Struktur:

Stem-Struktur ist die gesonderte Bezeichnung für eine Hybridisierungs-Struktur innerhalb der Triplex-Struktur, welche sich ausschließlich zwischen den Sonden des Sondenpaares ausbildet (Figur 1).

### Optimale Hybridisierung der Sonden:

Unter einer optimalen Hybridisierung der Sonden an das Target versteht man die Ausbildung einer komplexen Triplexstruktur zwischen Sondenpaar und Target in der Form, dass der Komplex soviel Stabilität aufweist, dass sich das erfindungsgemäße Verfahren laut Beschreibung durchführen lässt.

### Ausreichend komplementär:

Eine ausreichende Komplementarität zwischen den Sonden des Sondenpaares sowie zwischen dem Sondenpaar und dem Target liegt dann vor, wenn sich stabile Komplexe zwischen dem Sondenpaar und dem Target zur Ausführung des erfindungsgemäßen Verfahrens ausbilden.

### 3'-OH-Blockierun,a:

Ein in ein homogenes Detektionsverfahren inkorporiertes, als Sonde fungierendes Oligonukleotid hat nach dem Stand der Technik vorzugsweise keine Primer-Eigenschaften, d.h. das 3'-Ende der Sonde wird "blockiert" um zu verhindern, dass die Sonde in ein Primer-Extensionsprodukt (Amplikon) eingebaut wird. Eine "Blockierung" wird erreicht, indem entweder nicht-komplementäre Basen an das 3'-Ende der Sonde angehängt werden oder eine chemische Gruppe wie z.B. Biotin oder eine Phosphatgruppe an das 3'-Hydroxyl des letzten Nukleotids gekoppelt wird. Eine Blockierung kann weiterhin erreicht werden, indem das 3'-OH entfernt wird oder ein Nukleotid wie beispielsweise ein Didesoxynukleotid verwendet wird, dem generell die 3'-OH Gruppe fehlt.

### Tₘ-Wert:

Die Stabilität von hybridisierenden Nukleinsäureduplizes wird anhand der Schmelztemperatur oder "melting temperature" (Tₘ) gemessen. Der Tₘ einer spezifischen Nukleinsäureduplex-Struktur ist die Temperatur, bei der 50% der Basenpaarungen innerhalb einer Duplex dissoziiert vorliegen.

### Heteropolymere Zielsequenz:

Unter einer heteropolymeren Zielsequenz versteht man ein beliebiges Target mit einer beliebigen Abfolge von Basen oder Basenanaloga, die entweder einer natürlichen Sequenz wie genomischer DNA oder RNA, cDNA entspricht, oder semisynthetischer oder synthetischer Herkunft ist.

### Heteropolymeres Primerpaar:

Unter einem heteropolymeren Primerpaar versteht man ein Paar beliebiger Oligonukleotide mit einer beliebigen Abfolge von Basen oder Basenanaloga, die entweder natürlichen Ursprungs sind oder eine semisynthetische oder synthetischer Zielsequenz aufweisen oder synthetisch hergestellt werden und eine Spezifität für eine natürlichen Sequenz wie genomische DNA oder RNA, cDNA aufweisen. Ein Primer dienst als Initiationsstelle für die Synthese langer, komplementärer Nukleinsäurestränge, wenn er unter solchen katalysierenden Bedingungen eingesetzt wird, die die Synthese von der Zielnukleinsäure komplementären Primerextensionsprodukten gestatten. Diese Bedingungen liegen vor, wenn gleichzeitig verschiedene Desoxyribonukleosidtriphosphate und ein eine Polymerisierung induzierendes Agenz wie eine DNA Polymerase oder Reverse Transkriptase, ein Puffer mit geeignetem pH, Kofaktoren und Ionenstärke, und eine geeignete Reaktionstemperatur vorliegen.

### Mismatch:

Unter einem Mismatch versteht man mindestens eine Basenfehlpaarung zwischen zwei hybridisierten Nukleinsäuren.

## Patentansprüche

1. Verfahren zur Detektion von Ziel-Nukleinsäuren oder Nukleinsäureanaloga (Targets) oder zum Nachweis genetischer Polymorphismen, bei dem mindestens ein Paar markierter Oligonukleotide oder Oligonukleotidanaloga (Sonden) oder mindestens ein markiertes Sondenterzett eingesetzt wird, wobei die upstream Sonde(n) anteilig mit ihrem 5'-Ende an das Target und mit ihrem 3'-Ende anteilig an das 5'-Ende der downstream Sonde hybridisieren und das 3'-Ende der downstream Sonde wiederum anteilig an das Target hybridisiert, so dass im Resultat eine Triplex-Struktur zwischen der Ziel-Nukleinsäure und einem Sondenpaar ausgebildet wird, wobei
a) eine oder beide Sonden am 5'- und / oder am 3'-Ende eine Markierung besitzen
b) ein Mess-Signal nach partieller Hydrolyse des Sondenpaares durch ein im homogenen Assay enthaltenes Enzym generiert wird
c) eine ausreichende Hybridisierung der Sonden an das Target nur mit dem Sondenpaar oder Sondenterzett, nicht aber mit den Einzelsonden erfolgt
d) die anteilig zur Target-Hybridisierung befähigten Sequenzabschnitte der upstream und downstream Sonden an benachbarte Sequenzen des Targets hybridisieren und gleichzeitig zwischen dem 3'-Ende der upstream Sonde und dem 5'-Ende der downstream Sonde eine stabilisierende "Stem-Struktur" ausgebildet wird **dadurch gekennzeichnet, dass** die upstream Sonde mit je einem als Reporter und Quencher fungierenden Liganden markiert ist und keine 3'-OH-Blockierung aufweist, während die downstream Sonde unmarkiert und am 3'-OH blockiert ist.

2. Verfahren nach Anspruch 1 **dadurch gekennzeichnet, dass** als Markierungen Fluoreszenz- und/oder Quencherfarbstoffe eingesetzt werden.

3. Verfahren nach Anspruch 1 **dadurch gekennzeichnet, dass** als Enzym eine Polymerase dient.

4. Verfahren nach Anspruch 1 und 2, **dadurch gekennzeichnet, dass** das Sondenterzett aus 2 mit unterschiedlichen Liganden markierten upstream oder downstream Sonden besteht, die sich in der Sequenz um mindestens 1 Base unterscheiden und einer zugehörigen upstream oder downstream Sonde, die zur Ausbildung eines Stems mit den im Stembereich komplementären, dem Sondenterzett angehörenden, upstream oder downstream Sonden befähigt ist, wobei immer nur dann eine hydrolysierbare Triplex-Struktur zwischen upstream Sonde, downstream Sonde und Target gebildet wird, wenn eine ausreichende Komplementarität zwischen dem targethybridisierenden Sondenabschnitt mindestens einer der Sonden und dem Target besteht.

5. Verfahren nach Anspruch 5, **dadurch gekennzeichnet, dass** beim Sondenterzett die beiden upstream Sonden mit zwei, bei unterschiedlichen Wellenlängen emittierenden, als Reporter fungierenden Liganden sowie einem als Quencher fungierenden Liganden markiert sind und keine 3'-OH-Blockierung aufweisen, während die downstream Sonde unmarkiert und am 3'-OH blockiert ist.

## Claims

1. A method for detecting target nucleic acids or nucleic acid analogs (targets) or for detecting genetic polymorphisms, where at least one pair of marked oligonucleotides or oligonucleotide analogs (probes) or at least a marked probe trio are used, with the upstream probe(s) being proportionally hybridised by the 5' end thereof to the target and by the 3' end thereof to the 5' end of the downstream probe, and the 3' end of the downstream probe being proportionally hybridised to the target in such a way that a triplex structure is formed as a result between the target nucleic acid and a probe pair, with
a) one or both probes having a marking on the 5' and/or 3' end
b) a measuring signal being generated after partial hydrolysis of the probe pair by an enzyme contained in the homogeneous assay
c) sufficient hybridisation of the probes to the target occurring only with the probe pair or the probe trio but not with the individual probes
d) the sequence sections of the upstream and downstream probes, capable of proportional target hybridisation, hybridising to adjacent sequences of the target, and simultaneously a stabilising stem structure being formed between the 3' end of the upstream probe and the 5' end of the downstream probe,
**characterised in that** the upstream probe is marked with a ligand acting each as a reporter and a quencher, and does not comprise a 3' OH blocking whereas the downstream probe is unmarked and blocked on the 3' OH.

2. The method according to claim 1, **characterised in that** as markings fluorescent and/or quencher dyes are used.

3. The method according to claim 1, **characterised in that** a polymerase serves as an enzyme.

4. The method according to claim 1 and 2, **characterised in that** the probe trio is comprised of 2 upstream or downstream probes marked with different ligands, which probes differ in sequence by at least 1 base, and a related upstream or downstream probe, which is capable of forming a stem with the upstream or downstream probes complementary in the stem area and belonging to the probe trio, with a hydrolysable triplex structure between upstream probe, downstream probe and target being formed only, if sufficient complementarity exists between the target hybridising probe section of at least one of the probes and the target.

5. The method according to claim 5, **characterised in that** on the probe trio the two upstream probes are marked with two ligands emitting at different wave lengths and acting as reporters, as well as a ligand acting as a quencher and do not comprise any 3' OH blockings whereas the downstream probe is unmarked and blocked on the 3' OH.

## Revendications

1. Procédé pour la détection des acides nucléiques cibles ou des analogues d'acides nucléiques (cibles) ou pour la détection des polymorphismes génétiques en utilisant au moins une paire des oligonucléotides marqués ou des analogues d'oligonucléotides (sondes) ou au moins un trio de sondes marqués, la (les) sonde(s) en amont hybridisant proportionnellement avec l'extrémité 5' à la cible et avec l'extrémité 3' proportionnellement à l'extrémité 5' de la sonde en aval et l'extrémité 3' de la sonde en aval hybridisant proportionnellement à la cible de manière que de ce fait une structure triplex est formée entre l'acide nucléique cible et une paire de sondes,
a) une ou les deux sondes possédant un marquage à l'extrémité 5' et/ou 3'
b) un signal de mesure étant généré après une hydrolyse partielle de la paire de sondes par une enzyme contenue dans l'essai homogène
c) une hybridation suffisante des sondes à la cible ayant lieu seulement avec la paire de sondes ou le trio de sondes mais non pas avec les sondes individuelles
d) les parties de séquence des sondes en amont et en aval qualifiées proportionellement à la hybridation de cible hybridisant à des séquences adjacentes de la cible, et en même temps une structure "stem" stabilisante étant formé entre l'extrémité 3' de la sonde en amont et l'extrémité 5' de la sonde en aval,
**caractérisé en ce que** la sonde en amont est marqué avec un ligand agissant chacun en tant que rapporteur et quencher et ne comprend pas un blocage 3'-OH tandis que la sonde en aval est non marquée et bloquée à 3-OH.

2. Procédé selon la revendication 1, **caractérisé en ce qu'**en tant que des marquages des colorants fluorescents et/ou de colorants de quencher sont utilisés.

3. Procédé selon la revendication 1, **caractérisé en ce qu'**un polymérase sert en tant qu'une enzyme.

4. Procédé selon la revendication 1 et 2, **caractérisé en ce que** le trio de sondes comprend 2 sondes en amont ou en aval marquées avec des ligands différents qui diffèrent en séquence par au moins 1 base et une sonde associée en amont ou en aval qualifiée à former un "stem" avec les sondes en amont ou en aval complémentaires dans la région de "stem" et appartenant au trio de sondes, une structure triplex hydrolysable étant formé entre la sonde en amont, la sonde en aval et la cible, seulement si une complémentarité suffisante existe entre la partie de sonde hybridisant la cible d'au moins une des sondes et la cible.

5. Procédé selon la revendication 5, **caractérisé en ce qu'**au trio de sondes les deux sondes en amont sont marquées avec deux ligands émettant avec des longueurs d'onde différentes et agissant en tant que rapporteurs ainsi qu'un ligand agissant en tant que quencher et ne comprennent pas un blocage 3'-OH tandis que la sonde en aval est non marquée et bloquée à 3'-OH.
